# FASCICULE DE BREVET EUROPEEN

(11) **EP 1 475 082 B1**
(45) Date de publication et mention de la délivrance du brevet: **29.04.2009**
(21) Numéro de dépôt: 04291135.4
(22) Date de dépôt: 04.05.2004
(51) Int. Cl.: A61Q 3/02, A61K 8/26

(54) **Composition cosmétique à base de phyllosicates exfoliés**
Kosmetische Zusammensetzung enthaltend abblätternde Schichtsilicate
Cosmetic composition based on exfoliated phyllosilicates

(30) Priorité: 05.05.2003 FR 0305446
(43) Date de publication de la demande: 10.11.2004
(73) Titulaire: L'ORÉAL, 75008 Paris (FR)
(72) Inventeur: Mondet, Jean, 93600 Aulnay Sous Bois (FR); Ilekti, Philippe, 75003 Paris (FR)
(74) Mandataire: Le Coupanec, Pascale A.M.P.

(56) Documents cités:
- EP-A- 1 038 834
- EP-A- 1 203 789
- WO-A-03/099344
- US-A- 5 721 306
- US-A- 5 837 763
- US-B2- 6 500 411
- PATENT ABSTRACTS OF JAPAN vol. 017, no. 216 (C-1053), 28 avril 1993 (1993-04-28) & JP 04 357108 A (NIPPON PAINT CO LTD), 10 décembre 1992 (1992-12-10)
- PATENT ABSTRACTS OF JAPAN vol. 1996, no. 09, 30 septembre 1996 (1996-09-30) & JP 08 119844 A (KUNIMINE KOGYO KK), 14 mai 1996 (1996-05-14)

## Description

La présente invention concerne une composition cosmétique épaissie voire gélifiée pour le soin et/ou le maquillage des phanères et dont les dépôts peuvent notamment être translucides ou transparents. Ladite composition peut être colorée, translucide ou transparente.

Plus particulièrement, les compositions considérées selon l'invention, peuvent constituer un produit de maquillage des phanères, en particulier des ongles, ayant notamment des propriétés de soin et/ou de traitement non thérapeutique. Les produits cosmétiques plus particulièrement concernés sont par exemple, vernis à ongles, produits de soin des ongles.

D'une manière générale les compositions cosmétiques que ce soit celles destinées au maquillage de la peau comme les fonds de teint, rouges à lèvres, fards à paupières, fards à joues ou des phanères comme les mascaras, ou les vernis à ongles visent avant tout à procurer au niveau du support de maquillage, un effet esthétique. L'effet le plus souvent recherché est un effet coloré, de brillance et/ou de matité plus particulièrement dans le cas de fond de teint, ce dernier visant notamment à masquer efficacement les imperfections cutanées de type tels que microreliefs, rides, ridules, pores ou variations de couleurs.

Classiquement, les compositions de maquillage précitées sont appliquées sur la surface à maquiller sous la forme d'un dépôt destiné à procurer les effets de coloration et/ou de brillance et/ou de matité attendus.

Or, l'utilisateur cherche de plus en plus à concilier avec les effets précédents un effet dit naturel. Le maquillage appliqué, notamment au niveau de la peau, doit procurer l'effet esthétique recherché à savoir coloré, brillant et/ou mat tout en conférant l'illusion d'un aspect naturel au support de maquillage. En d'autres termes, il serait souhaitable que le dépôt de maquillage soit invisible ou encore que la composition cosmétique de maquillage et/ou de soin soit, après application en fine couche, parfaitement transparente ou bien suffisamment translucide pour préserver un effet naturel de la peau.

Des compositions de maquillage colorées, et dites transparentes ou translucides, et dont le dépôt sur le support de maquillage est transparent ou translucide, ont déjà proposées. Ces compositions possèdent généralement une phase grasse liquide ou une phase organique non miscible à l'eau structurée par un agent de texture autre qu'une cire ou charge organique ou inorganique conventionnelle qui présentent précisément l'inconvénient d'opacifier la composition cosmétique. Dans le cas particulier des charges, cet effet indésirable est notamment lié à leur taille, généralement à l'échelle du micron, et/ou à leur matité naturelle.

Les nouveaux agents structurants proposés sont généralement de nature organique à savoir des polymères (EP 1 068 856), des polyamides gélifiants (WO 00/247627), des esters ou amides de N-acylaminoacides (WO 01/97758), ou des dérivés siliconés (WO 97/36573 ; WO 02/17870, WO 02/17871, US 6 051 216). Toutefois, l'utilisation de ces composés ne donne pas entière satisfaction. En particulier, certains d'entre eux confèrent un caractère collant, bien entendu indésirable aux compositions cosmétiques correspondantes.

Le document JP 4 357 108 concerne un additif utile à l'état de gel ou pulvérulent et particulièrement utile en tant qu'agent d'épaississement d'un revêtement par le traitement d'une argile minérale avec un tensioactif cationique, de manière à rendre l'intérieur de couches lipophile et en y rajoutant ensuite une substance lipophile.

De manière surprenante, les inventeurs ont découvert que l'association de phyllosilicates, notamment dérivant de phyllosilicates intercalés, à des ingrédients cosmétiques permet l'obtention d'une composition épaissie, voire gélifiée et dont le dépôt ou film sur le support de maquillage peut être translucide ou transparent, tout en cumulant de bonnes propriétés de texture, de tenue, de brillance, de matité et/ou d'absence de migration.

Ces phyllosilicates intercalés constituent donc un additif particulièrement intéressant dans le domaine de la cosmétique pour accéder à des dépôts de compositions cosmétiques, notamment translucides ou transparents et qui s'avèrent en outre avantageusement dotés de bonnes propriétés de tenue dans le temps et dénués de tout caractère collant.

Les phyllosilicates, plus communément appelés argiles, désignent d'une manière générale des silicates en feuillets.

Le terme « phyllosilicates intercalés », signifie que des phyllosilicates ont été traités avec des composés organiques ou inorganiques en vue d'introduire des molécules de ces composés dans les espaces interfoliaires desdits phyllosilicates, d'une part pour augmenter la distance entre les feuillets et, d'autre part pour leur apporter un caractère organophile. Généralement, une quantité suffisante de molécules est adsorbée entre deux feuillets adjacents de phyllosilicates de manière à augmenter leur espace interfoliaire à une dimension d'au moins 5 angströms, en particulier d'environ 10 angströms et à favoriser ainsi l'exfoliation consécutive du matériau intercalé sous la forme de feuillets séparés. Cette exfoliation, ou encore délamination, est généralement réalisée sous cisaillement en présence d'un solvant organique ou dans une matrice polymérique de manière à obtenir des « phyllosilicates exfoliés ».

A ce jour, deux techniques d'intercalation sont plus particulièrement utilisées.

La première technique notamment décrite dans le document WO 93/04118 consiste à échanger les cations minéraux hydrophiles, originellement présents entre les feuillets de phyllosilicates, par des cations « onium » organiques, généralement des alkylammoniums quaternaires. Les phyllosilicates ainsi intercalés sont utilisés sous leur forme exfoliée dans des matrices polymériques en vue de renforcer notamment leurs propriétés mécaniques. Les cations d'ammonium quaternaire sont en particulier bien connus pour convertir des phyllosilicates très hydrophiles tels que les montmorillonites de calcium ou de sodium en phyllosilicates organophiles.

La seconde technique, vise à modifier la nature des ligands auxquels sont coordonnés les cations des espaces interfoliaires. A l'état naturel, les cations des espaces interfoliaires sont coordonnés à des molécules d'eau. Cette seconde méthode vise à substituer à ces molécules d'eau des molécules hydrocarbonées organiques spécifiques comportant au moins un groupe polaire. Ainsi, le brevet US 5 721 306 propose plus particulièrement à titre d'agent intercalant des molécules hydrocarbonées possédant au moins un groupement polaire de type hydroxyle, carbonyle, carboxyle, amine, amide, éther, ester, et voire aussi sulfate, sulfonate, sulfinate, sulfamate, phosphate, phosphonate ou phosphinate, ou un groupe aromatique, capable d'interagir avec les cations métalliques liés aux surfaces des feuillets de phyllosilicates. Il s'agit notamment de dérivés d'alkylpyrrolidone, de polyvinylpyrrolidone, de polyvinylalcool, de polyoxyalkylènes, de polyamide ou de polyimide. Les phyllosilicates ainsi intercalés, sont exfoliés ultérieurement. Ils sont notamment proposés dans ce document, comme agent épaississant pour préparer des formulations de type gel thixotropique visqueux, voire de gels solides, et en particulier à titre de véhicules pour une grande variété de matières actives. Plus récemment, le brevet US 6 500 411, propose d'utiliser des phyllosilicates intercalés et exfoliés à l'aide de dérivés polyphénoliques naturels, à l'image de la lignine par exemple, dans des compositions cosmétiques aqueuses notamment de type formulation solaires.

La présente invention concerne, selon l'un de ses premiers aspects une composition cosmétique de vernis à ongles comprenant un milieu physiologiquement acceptable contenant:
- au moins une phase grasse liquide ou
- une phase organique non miscible à l'eau, ladite phase organique comprenant un ou plusieurs solvants organiques dont la miscibilité dans l'eau à 25°C est inférieure ou égale à 50% en poids et
ladite phase étant structurée par au moins une quantité efficace de phyllosilicate exfolié dérivant d'au moins un phyllosilicate intercalé.

Dans l'expression «phyllosilicates exfoliés dérivant d'au moins un phyllosilicate intercalé », on entend par le terme « dérivant », qualifié le fait que les phyllosilicates à l'état de feuillets sont obtenus à partir de phyllosilicates intercalés par application à ces derniers d'une force de cisaillement suffisante pour permettre au moins en partie, et notamment en totalité leur exfoliation. Le type de force de cisaillement susceptible d'être appliquée est plus particulièrement détaillé ci-après.

Selon un mode de réalisation particulier de l'invention, le milieu physiologiquement acceptable contient au moins une phase grasse liquide.

En particulier, la composition est une émulsion.

De manière avantageuse, les émulsions contenant les phyllosilicates exfoliés conformes à l'invention peuvent contenir une moindre quantité d'agents émulsionnants et d'agents co-émulsionnants, voire être totalement exemptes de ces agents.

Les inventeurs ont ainsi constaté que les phyllosilicates conformes à l'invention pouvaient être avantageusement mis à profit à titre d'agent stabilisant pour assurer la stabilité d'émulsions cosmétiques, qui d'ordinaire requiert une quantité efficace en tensioactifs(s) ou en agents émulsionnants et le cas échéant en agents co-émulsionnants. Les phyllosilicates permettent de réduire significativement, voire de s'affranchir totalement de la présence de ces tensioactifs classiques, ou agents émulsionnants et co-émulsionnants, usuellement requis avec un avantage évident sur le plan de l'innocuité.

Avantageusement, les compositions selon l'invention peuvent être transparentes ou translucides et/ou susceptibles de donner un dépôt transparent ou translucide.

Au sens de l'invention, cette propriété de transparence ou de translucidité « dans la masse » signifie qu'une couche de la composition d'une épaisseur donnée, laisse passer une partie de la lumière visible. Si cette partie de la lumière visible est diffusée, la composition sera définie comme étant une composition translucide dans la masse, et si, au contraire, elle n'est pas diffusée, alors la composition sera définie comme étant une composition transparente dans la masse.

La présente invention a également pour objet, selon un autre de ses aspects, l'utilisation de phyllosilicate(s) exfolié(s) conforme(s) à l'invention à titre d'agent pour texturer la phase grasse liquide et/ou la phase organique non miscible à l'eau d'une composition cosmétique de soin et/ou de maquillage des phanères, notamment de type émulsion.

La présente invention a encore pour objet, selon un autre de ses aspects, l'utilisation de phyllosilicates exfoliés conformes à l'invention pour stabiliser des émulsions notamment phase aqueuse-dans-phase huileuse ou phase huileuse-dans-phase aqueuse.

Dans le cadre de la présente invention, les phyllosilicates exfoliés sont dénués de toute activité de véhicule à l'égard de tout actif susceptible d'être présent dans ladite composition cosmétique. En d'autres termes, ils n'ont pas vocation à participer au conditionnement d'une substance active dans la composition cosmétique.

La présente invention a encore pour objet, un procédé de maquillage des phanères comprenant l'application sur les phanères d'au moins une composition conforme à la présente invention.

Selon un autre de ses aspects, l'invention a encore pour objet un support synthétique, sur lequel est présent en tout ou partie de sa surface au moins une couche d'une composition selon l'invention.

### TRANSLUCIDITE ET TRANSPARENCE

Les compositions cosmétiques de l'invention peuvent avantageusement procurer une transparence ou une translucidité au niveau de leur dépôt sur un support de maquillage et/ou d'autre part, posséder en soi un aspect translucide ou transparent avant application, dit « translucide ou transparent dans le pot » ou encore, « translucide ou transparent dans la masse ».

Plus précisément, la propriété de transparence ou de translucidité de la composition selon l'invention est déterminée de la façon suivante : on coule la composition à tester dans un pot Volga 30ml, on laisse la composition refroidir (pendant 24h à température ambiante) et on place en dessous une feuille blanche sur laquelle est tracée au feutre noir une croix d'environ 2 mm d'épaisseur. Si la croix est visible à l'oeil nu à la lumière du jour à une distance d'observation de 40 cm, la composition est transparente ou translucide.

Toutefois, le caractère transparent ou translucide d'une composition cosmétique, par exemple en stick, et qui plus est, du dépôt obtenu à partir de celle-ci, est extrêmement difficile à prévoir. Ainsi, il n'est absolument pas possible de prédire à l'avance quel composant particulier et en quelle proportion, permettra d'obtenir la transparence ou la translucidité de la composition et, a fortiori, celle du dépôt.

La formulation de compositions cosmétiques transparentes ou translucides est donc totalement aléatoire et ne peut en aucun cas se déduire, à partir de formulations déjà existantes ou des propriétés, par exemple des propriétés optiques, des composés rentrant dans ces compositions.

### PHYLLOSILICATES EXFOLIES

Comme précisé précédemment, les phyllosilicates exfoliés dérivant ou encore obtenus à partir d'au moins un phyllosilicate intercalé peuvent être présents dans la composition cosmétique selon l'invention sous divers degrés d'exfoliation.

Ainsi certains phyllosilicates exfoliés peuvent être présents sous une forme totalement exfoliée c'est-à-dire sous la forme d'un unique feuillet et d'autres en revanche sous une forme partiellement exfoliée c'est-à-dire sous une forme comprenant, deux ou plus, feuillets encore associés. Avantageusement, au moins 50 %, notamment 70 %, en particulier 90 %, voire 95 % de ces phyllosilicates exfoliés peuvent être formés de moins de 10 feuillets, notamment moins de 5 feuillets, en particulier moins de 3 feuillets, voire d'un seul feuillet de phyllosilicate.

L'ensemble de ces formes peut bien entendu coexister dans la composition cosmétique selon l'invention voire en mélange avec des phyllosilicates intercalés non exfoliés.

Le phyllosilicate exfolié peut être soit préparé par exfoliation de phyllosilicate intercalé, puis être incorporé dans la composition cosmétique, soit incorporé dans la composition cosmétique sous la forme de phyllosilicate intercalé et y être ensuite exfolié.

Les phyllosilicates exfoliés selon l'invention peuvent être caractérisés par un facteur de forme qui est le rapport de la plus grande dimension sur la plus petite dimension. Ce facteur de forme peut varier de 50 à 2000, notamment de 75 à 1500, et en particulier de 200 à 1000.

Les phyllosilicates exfoliés selon l'invention peuvent présenter une épaisseur moyenne supérieure à 5 Å et une épaisseur maximum inférieure à 100 Å, et en particulier variant de 10 Å à 50 Å.

Compte tenu de leur faible épaisseur, à l'échelle de quelques angströms, les phyllosilicates exfoliés, avantageusement, n'apportent pas d'opacité contrairement aux charges conventionnelles. A l'image des compositions matifiantes classiques, les compositions selon l'invention et notamment celles destinées à être appliquées sur la peau, comme le fond de teint, s'avèrent avantageusement efficaces pour unifier le teint et notamment estomper les imperfections cutanées, tout en procurant un aspect naturel à la peau maquillée grâce à la translucidité ou transparence de leur dépôt. De telles compositions possèdent un bon pouvoir diffusant de la lumière en surface et stable dans le temps. Ces phyllosilicates permettent par ailleurs de conférer à une composition cosmétique des propriétés intéressantes en terme de tenue et d'absence de migration sans pour autant altérer ses qualités esthétiques telles qu'effet coloré et brillance.

De même, les phyllosilicates peuvent être utilisés pour assurer la stabilisation d'émulsion et, à ce titre, être utilisés efficacement en substitution et/ou remplacement total ou partiel des tensioactifs généralement requis pour assurer ce type de stabilisation.

Les phyllosilicates utilisables selon l'invention, dérivent plus particulièrement phyllosilicates de type smectite comme les montmorillonites notamment les montmorillonites de sodium, de potassium et/ou de calcium, les nontronites, beidellites, volkonskoïtes, hectorites, saponites, sauconites, sobockites, stevensites, svinfordites, vermiculites et leurs mélanges. Conviennent tout particulièrement à l'invention les phyllosilicates de type montmorillonite.

Les phyllosilicates exfoliés selon l'invention présentent avantageusement, adsorbés à la surface de leurs feuillets, au moins un « agent intercalant », lequel agent était intercalé entre les feuillets du phyllosilicate dit « intercalé » utilisé pour préparer la composition selon l'invention. Les phyllosilicates exfoliés possèdent en particulier au moins 15 % en poids, notamment au moins 20 % en poids et plus particulièrement au moins 30 % en poids d'agent intercalant par rapport au poids de phyllosilicate sec (comprenant moins de 5 % d'eau).

Les agents intercalants peuvent être adsorbés sur tout ou partie de la surface des feuillets de phyllosilicate. Les agents intercalants peuvent notamment présenter avec ledit phyllosilicate ou avec des cations présents à la surface de ses feuillets, des liaisons de type hydrogène, ionique, covalente et/ou des interactions hydrophiles de Van der Waals.

D'une manière générale, les agents intercalants considérés selon l'invention possèdent dans leur structure chimique au moins un groupement hydrophobe, notamment une chaîne alkyle en C₄ à C₅₀, alkylène en C₄ à C₅₀ et/ou alkylaryle en C₄ à C₅₀. En revanche, les phyllosilicates exfoliés selon l'invention sont avantageusement dépourvus à la surface de leurs feuillets d'agent intercalant polyphénolique.

Dans le cadre de la présente invention, les agents intercalants plus particulièrement considérés sont des composés choisis parmi :
- les oniums possédant au moins une chaîne hydrocarbonée en C₁-C₅₀, notamment en C₄-C₅₀, et
- les composés à groupe polaire comme les composés organiques, de nature polymérique ou non, possédant au moins un cycle aromatique ou au moins un groupe polaire choisi parmi les groupes carbonyle, hydroxyle, polyol (dont glycol, glycerol, etc.), acide carboxylique, aldéhyde, cétone, amine, amide (linéaire ou cyclique en particulier pyrrolidone, caprolactame), ester, lactone, éther, voire sulfate, sulfonate, sulfinate, sulfamate, phosphate, phosphonate et phosphinate.

Comme indiqué précédemment, les agents intercalants du type oniums remplacent en partie ou totalement les ions minéraux hydrophiles (Na+, K+, ...) présents dans les phyllosilicates à l'état naturel. On parle encore d'intercalation par échange d'ions. Quant aux agents intercalants possédant au moins un groupe polaire, ils remplacent en partie ou totalement les molécules d'eau qui coordonnaient initialement les cations métalliques hydrophiles présents entre les feuillets.

### AGENT INTERCALANT DE TYPE ONIUM

Par « oniums », on désigne les groupes ammonium, sulfonium ou phosphonium tel qu'il est indiqué par exemple dans la demande WO 93/04118 de ALLIED SIGNAL.

Parmi ces oniums, on peut citer plus particulièrement les ammoniums primaires, les ammoniums secondaires, les ammoniums tertiaires notamment de type N⁺H₃R₁, N⁺H₂R₁R₂ et N⁺HR₁R₂R₃ et les ammoniums quaternaires N⁺R₁R₂R₃R₄ dans lesquels les groupes R₁, R₂, R₃, R₄, égaux ou différents représentent des chaînes hydrocarbonées en C₁-C₅₀, dont une au moins est plus particulièrement en C₄-C₅₀, ces chaînes hydrocarbonées pouvant être linéaires, ramifiées ou cycliques, saturées ou insaturées et pouvant comporter un ou plusieurs hétéroatomes tels que O, S, N, Si ou P. Il peut notamment s'agit de chaînes alkyle en C₄ à C₅₀, alkylène en C₄ à C₅₀ ou alkylaryle en C₄ à C₅₀.

Selon un aspect de la présente invention, l'un des substituants R₁ à R₄ du groupe omnium peut être de nature aromatique (benzyle ou phényle) ou alkyl aryle, les autres groupes étant alors des chaînes alkyles en C₄ à C₅₀ comme défini précédemment.

A titre illustratif et non limitatif des agents intercalants de type oniums utilisables selon l'invention, on peut notamment citer ceux décrits dans la demande WO 93/04118 de ALLIED SIGNAL, les agents intercalants di- ou multi- oniums décrits dans EP 1 038 834 et WO 00/09605 de AMCOL et les agents intercalants à groupe onium, de préférence ammoniums primaires, secondaires ou tertiaires possédant deux chaînes alkyles en C ≥ 10 tels que décrits dans JP 04 357108 de NIPPON PAINT.

Parmi ces oniums, conviennent tout particulièrement les ammoniums primaires, secondaires, tertiaires ou quaternaires, de préférence quaternaires et possédant au moins une chaîne alkyle en C₄ à C₁₀, chaîne qui est de préférence linéaire ou ramifiée et de préférence saturée, comme par exemple une chaîne butyle, isobutyle, pentyle, isopentyle, hexyle, heptyle, éthyl 2-hexyle, octyle, nonyle, décyle, undécyle, dodécyle, ....octadécyle. Parmi ces cations ammoniums, conviennent tout particulièrement ceux qui possèdent au moins une chaîne alkyle de type dodécyle ou octadécyle.

### AGENTS INTERCALANTS A GROUPE POLAIRE

Parmi les agents intercalants à groupe polaire, on distinguera
a) ceux qui ne sont pas de nature polymérique,
b) ceux qui sont de nature polymérique.

Au sens de la présente invention, on désigne sous le terme de composé de nature polymérique, un composé possédant au moins deux motifs de répétition, notamment au moins trois motifs de répétition, en particulier au moins dix motifs de répétition voire au moins quinze motifs de répétition. Il peut être par ailleurs composé d'un unique motif répétitif (homopolymère) ou d'au moins deux motifs répétitifs de nature différente (copolymère).

### a) Agents intercalants à groupe polaire de nature non polymérique

Ils comportent au moins un groupe polaire tel que défini ci-dessus et au moins, de préférence une chaîne hydrophobe notamment hydrocarbonée en C₄-C₅₀, qui peut être linéaire, ramifiée ou cyclique, saturée ou non et peut en plus contenir des hétéroatomes tels que O, S, N, Si ou P. Il peut s'agir notamment d'une chaîne alkyle en C₄-C₅₀, alkylène en C₄-C₅₀ ou alkyle aryle en C₄-C₅₀. De tels composés sont notamment décrits dans les documents US 5 721 306, EP 780 340 et US 6 242 500.

A titre illustratif de ce type de composé, on peut notamment citer ceux comprenant à titre de groupe polaire, au moins un groupe choisi parmi les groupes alcools C₆₋₂₄, les glycérols ayant au moins une chaîne en C₆-C₂₄, les acides carboxyliques en C₆-C₂₄ notamment ceux décrits dans EP 780 340, les groupes amides, de préférence cycliques à l'image des lactames tels que les pyrrolidone ou caprolactame et substitués par un groupe aromatique, comme ceux décrits dans US 6 242 500, ou par une chaîne alkyle en C₄-C₅₀, de préférence en C₈-C₃₀, de préférence en C₁₂-C₂₅ comme par exemple les alkylpyrrolidones avec une chaîne alkyle en C₄-C₅₀, en particulier en C₈-C₃₀. Il peut notamment s'agir de la dodécyl pyrrolidone décrite en particulier dans *« Nanocomposites produces utilizing a novel dipole clay surface modification in polymer-clay composites* », BEALL G.W., Chemistry and Technology of Polymer Additives (1999), 266-280, Editor : Al-Malaika, Sahar ; Publisher : Blackwell, Oxford UK.

### b) Agents intercalants à groupe polaire de nature polymérique

Il s'agit plus particulièrement d'oligomères homo- ou co-polymères synthétiques qui comportent au moins un noyau aromatique ou un groupe polaire tel que défini ci-dessus. Le PM peut varier de 300 à 200 000, et en particulier de 500 à 40 000 ; De tels agents sont notamment décrits dans US 5 837 763.

Ces oligomères ou polymères peuvent être hydrophiles ou hydrophobes.

A titre représentatif de ces agents intercalants polymériques hydrophiles, on peut notamment citer les dérivés polyvinylpyrrolidones (PVP), les dérivés polyvinylalcools (PVA), notamment lorsque ceux-ci sont quasiment sous leur forme acétate de polyvinyle hydrolysé ou en d'autres termes possèdent moins de 5 % de groupes acétyles résiduels, les dérivés polyacryliques sous leur forme polymérique et copolymérique et plus particulièrement sous forme de leurs sels métalliques, les dérivés acides polyméthacryliques (PMAA), les dérivés polyvinyloxazolidones (PVO) et polyvinylméthyloxazolidones (PVMO), les dérivés polyvinyloxazolines.

Comme autres agents intercalants polymériques hydrophiles, on peut citer également les copolymères des motifs cités ci-dessus, copolymères entre ces mêmes motifs ou copolymères avec d'autres monomères hydrophiles ou fortement polaires tels que : (méth)acrylate d'hydroxyéthyle, (méth)acrylate de 2 hydroxypropyle, (méth)acrylate de méthyle, acétate de vinyle, (méth)acrylamide, NN diméthyl acrylamide, acide crotonique, anhydride maléique, méthyl vinyl éther.

Les agents intercalants polymériques peuvent également être organophiles ou même lipophiles à condition de posséder au moins un, voire plusieurs groupe(s) polaire(s), tel(s) que défini(s) précédemment. On retiendra en particulier les copolymères obtenus par réaction entre un monomère polaire et hydrophile tel que choisi ci-dessus (vinyl pyrrolidone, vinyl oxazoline, vinyl oxazolidone, alcool vinylique, acide (méth)acrylique) avec au moins un monomère plus organophile voire lipophile tel que les esters d'acide (méth)acrylique tels (méth)acrylates d'éthyle, de butyle, d'isobutyle, de tertiobutyle, d'hexyle, de cyclohexyle, d'octyle, de 2 éthylhexyle, nonyle, décyle, undécyle, dodécyle, ... octadécyle, béhényle, les esters vinyliques tels que propianate, versatate, benzoate ; les (méth)acrylamides tels que diacétone acrylamide, butyl (méth)acrylamide, les tertiobutyl (méth)acrylamide, tertiohexyl (méth)acrylamide, tertiooctyl (méth)acrylamide ; les oléfines telles que éthylène, propylène, butène, isobutène, hexène, octène, dodecène, octadécène, eicosène, le styrène et les styrènes substitués.

D'autres polyols polymériques et alcools polyhydriques, hydrosolubles ou hydrophiles tels que les polysaccharides sont également susceptibles de constituer des agents intercalants polymériques.

Certaines associations phyllosilicates/agent intercalant telles que montmorillonite-PVP, montmorillonite-PVA, et montmorillonite-alkylpyrrolidone comme par exemple la montmorillonite-dodécylpyrrolidone sont particulièrement avantageuses dans le cadre de la présente invention.

L'intercalation des phyllosilicates, considérés dans le cadre de la présente invention, peut être réalisée selon des protocoles classiques tels que ceux notamment décrits dans le brevet US 5 721 306 et le document WO 93/04118.

En l'occurrence, les agents intercalants peuvent être introduits ou adsorbés à l'intérieur des espaces interfoliaires du phyllosilicate selon le mode de réalisation suivant : l'intercalation est réalisée en mélangeant intimement les phyllosilicates par extrusion ou agitation à hélice de manière à former une composition intercalante comprenant le phyllosilicate dans un polymère intercalant, une solution aqueuse d'agent intercalant ou une solution organique d'agent intercalant. Pour obtenir une intercalation suffisante en vue de l'exfoliation, l'agent intercalant est généralement mis en présence du phyllosilicate dans la composition d'intercalation à raison d'un rapport pondéral agent intercalant/phyllosilicate d'au moins environ 1/20, notamment d'au moins environ 1/10, et plus particulièrement d'environ 1/5, voire d'environ 1/4, de manière à obtenir une intercalation efficace de l'agent entre des feuillets adjacents de phyllosilicate. L'espace interfoliaire peut être ainsi augmenté de 10 à 100 angstroms pour garantir une exfoliation consécutive, facile et totale. Le véhicule d'intercalation, de préférence l'eau, le cas échéant en mélange avec un solvant organique, peut être introduit après pré-solubilisation ou prédispersion de l'agent intercalant dans le véhicule, ou être directement mélangé avec l'agent intercalant sec et le phyllosilicate sec L'agent intercalant est généralement présent en proportion à raison d'au moins 15 % en poids, notamment d'au moins 20 % en poids et plus particulièrement d'au moins 30 % en poids par rapport au poids de phyllosilicate sec (comprenant moins de 5 % d'eau). Cette quantité peut notamment varier de 20 à 50 % en poids par rapport au poids de phyllosilicate à l'état sec.

Les agents intercalants polymères peuvent souvent être obtenus par polymérisation (homo ou copolymérisation) directe des monomère(s) préalablement intercalés entre les feuillets du phyllosilicate.

En ce qui concerne l'exfoliation des phyllosilicates ainsi intercalés, elle est réalisée de manière conventionnelle, généralement par application au milieu dans lequel sont dispersés les phyllosilicates intercalés, une vitesse de cisaillement suffisante pour provoquer la délamination recherchée. Cet aspect est plus particulièrement développé ci-après.

Il existe par ailleurs un certain nombre de phyllosilicates intercalés et exfoliés déjà disponibles commercialement.

A titre illustratif des phyllosilicates exfoliés convenant à l'invention, on peut plus particulièrement citer ceux commercialisés par :
a) la société NANOCOR aux Etats-Unis. Comme exemples d'argiles (type montmorillonite) intercalées par un alkyl ammonium quaternaire, c'est-à-dire par échange d'ions, on peut citer les NANOMER 1.24 T, I.30 TC et 1.34 TCN ; comme exemples d'argiles intercalées par un dérivé organique de pyrrolidone (technologie ion-dipole par échange de l'eau coordonnée) les NANOMER 1.35 K et 1.46 D, également décrits dans : « Advances in manomer additives for clay/polymer nanocomposites » LAN, T. (NANOCOR), Additives 99, International Conférence, 8th, San Francisco, March 22-24, 1999 (1999) Paper 12/1 - Paper 12/11, Publisher = Executive Conférence Management, Plymouth, Mic.
b) la société SOUTHERN CLAY PRODUCTS aux Etats-Unis, qui produit également des argiles intercalées par un alkyl ammonium quaternaire ex CLOISITE 25A et CLOISITE 30B cités dans l'article de POITTEVIN B., Polymer 43, 4017-23 (2002).

Les phyllosilicates exfoliés selon l'invention sont présents en une quantité efficace pour texturer ou encore pour rigidifier ou gélifier la phase grasse liquide ou phase organique non hydrosoluble de ladite composition.

En particulier, les phyllosilicates exfoliés peuvent être présents à raison de 0,05 à 30 % en poids, notamment de 0,1 à 20 % en poids et plus particulièrement de 0,5 à 10 % en poids dans la composition cosmétique.

Dans le cas où les argiles exfoliées sont mises en oeuvre à des fins de stabilisation d'émulsion, la quantité efficace est, pour des raisons évidentes, susceptible de varier significativement selon que ces argiles sont associées ou non à d'autre(s) tensioactif(s). L'homme de l'art est à même de procéder aux ajustements correspondants.

Ces phyllosilicates sont généralement directement introduits sous une forme exfoliée dans la composition. Toutefois, l'invention s'étend également aux compositions dans lesquelles les phyllosilicates exfoliés seraient générés *in situ,* par exemple par simple agitation de la composition cosmétique contenant des phyllosilicates intercalés c'est à dire sous une forme non exfoliée, voire du dispositif dans lequel est conditionnée ladite composition. Comme précisé précédemment, les phyllosilicates exfoliés selon l'invention peuvent être présents en mélange avec des phyllosilicates intercalés non exfoliés, de nature chimique identique ou non.

### PHASE GRASSE

La composition selon l'invention peut comprendre au moins une phase grasse liquide. En particulier, la phase grasse peut constituer la phase continue ou la phase dispersée d'une émulsion, multiple ou non, et notamment d'une microémulsion.

En particulier, la composition peut posséder par exemple une phase grasse continue, pouvant contenir moins de 10 % en poids d'eau, notamment moins de 5 % en poids d'eau, voire moins de 1 % en poids d'eau par rapport à son poids total, et en particulier peut être sous forme anhydre.

Par phase grasse liquide on entend, au sens de l'invention, une phase grasse liquide à température ambiante (25 °C) et à pression atmosphérique composée d'un ou plusieurs corps gras liquide à température ambiante de type huiles et compatibles entre eux.

Par phase grasse liquide structurée, on entend une phase grasse liquide rigidifiée ou gélifiée ou seulement épaissie.

Par phase grasse liquide rigidifiée, on entend que la phase grasse ne s'écoule pas sous l'effet de son propre poids lorsqu'on lui associe un phyllosilicate exfolié selon l'invention.

Par phase grasse liquide gélifiée ou épaissie, on entend que la viscosité de cette phase grasse est augmentée du fait de son association avec un phyllosilicate exfolié selon l'invention.

La phase grasse de la composition selon l'invention peut notamment comprendre, à titre de corps gras liquide, au moins une huile volatile ou non volatile ou un de leurs mélanges.

La phase huileuse de la composition selon l'invention peut être présente en proportion variant de 1 à 80 %, en particulier de 1 à 50 % en poids, par rapport au poids total de la composition.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permetyls^{®} , les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt^{®} par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la phase grasse selon l'invention à une teneur allant de 0,1 % à 98 % en poids, notamment de 1 % à 65 % en poids, et en particulier de 2 % à 50 % en poids, par rapport au poids total de la phase grasse.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique,
- les huiles siliconées de type polyméthylsiloxanes (PDMS) et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates.

Les huiles non volatiles peuvent être présentes dans la phase grasse liquide en une teneur allant de 0,01 à 90 % en poids, notamment de 0,1 % à 85% en poids, et en particulier de 1 % à 70 % en poids, par rapport au poids total de ladite phase.

Plus généralement, la phase grasse liquide à température ambiante et à pression atmosphérique peut être présente à raison de 0,01 à 99,5 % en poids, notamment de 0,5 à 85 % en poids, en particulier de 10 à 80 % en poids, voire de 20 à 75 % en poids par rapport au poids de la composition.

La composition selon l'invention peut également comprendre un corps gras solide à température ambiante et à pression atmosphérique tel que les cires, les composés pâteux, les gommes et leurs mélanges sous réserve bien entendu que ceux-ci n'affectent pas sa translucidité ou transparence ni celles de son dépôt sur le support de maquillage.

En ce qui concerne le corps gras solide à température ambiante et à pression atmosphérique, il peut être choisi parmi les cires, les corps gras pâteux, les gommes et leurs mélanges. Comme précisé précédemment, ce corps gras solide ne peut être présent qu'à une quantité compatible avec le caractère translucide ou transparent requis selon l'invention.

La cire peut être solide à température ambiante (25°C), à changement d'état solide/liquide réversible, ayant une température de fusion supérieure à 30°C pouvant aller jusqu'à 200°C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, et les cires microcristallines et leurs mélanges.

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

Par composé pâteux au sens de l'invention, on entend un composé ayant une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23 °C, sous la forme d'une fraction liquide et d'une fraction solide. En d'autres termes, la température de fusion commençante du composé pâteux est inférieure à 23°C. La fraction liquide du composé pâteux mesurée à 23 °C représente de 9 à 97 % en poids du composé. Cette fraction liquide à 23 °C représente de préférence de 15 à 85 %, de préférence encore de 40 à 85 % en poids par rapport au poids total du composé.

La fraction liquide en poids du composé pâteux à 23 °C est égale au rapport de l'enthalpie de fusion consommée à 23 °C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C, c'est-à-dire constitué d'une fraction liquide et d'une fraction solide.

La fraction liquide du composé pâteux mesurée à 32 °C représente de préférence de 30 à 100 % en poids du composé, de préférence de 80 à 100 %, de préférence encore de 90 à 100 % en poids du composé. Lorsque la fraction liquide du composé pâteux mesurée à 32 °C est égale à 100 %, la température de la fin de la plage de fusion du composé pâteux est inférieure ou égale à 32°C.

La fraction liquide du composé pâteux mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Plus particulièrement, ces corps gras peuvent être des composés hydrocarbonés, éventuellement de type polymérique; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503^{®} et DC25514^{®} et leurs mélanges.

### PHASE ORGANIOUE NON MISCIBLE A L'EAU

La composition selon l'invention peut comprendre une phase organique non miscible à l'eau. Cette phase organique non miscible peut être constituée d'un ou plusieurs solvants organiques non miscibles à l'eau.

Au sens de la présente invention, un solvant est considéré comme non miscible à l'eau à partir du moment où il manifeste une miscibilité dans l'eau inférieure ou égale à 50 % en poids, notamment inférieure ou égale à 30 % en poids voire inférieure ou égale à 20 % en poids et plus particulièrement inférieure ou égale à 10 % voire inférieure ou égale à 9 % en poids à 25 °C.

A titre représentatif de ces solvants organiques, volatils ou non à température ambiante, on peut plus particulièrement citer les esters à chaînes courtes (ayant de 4 à 8 atomes de carbone au total) tels que l'acétate d'éthyle, les acétates de propyle, de butyle, d'aryle, l'acétate d'isopentyle et leurs mélanges.

### PHASE AQUEUSE

La composition selon l'invention peut aussi comprendre un milieu aqueux, constituant une phase aqueuse, qui peut former la phase continue de la composition.

La phase aqueuse peut être constituée essentiellement d'eau. Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leurs mélanges.

La phase aqueuse (eau et éventuellement le solvant organique miscible à l'eau) peut être présente à une teneur allant de 1 à 95 % en poids, notamment allant de 3 à 80 % en poids, et en particulier allant de 5 à 60 % en poids, par rapport au poids total de la composition. Selon une variante particulière, elle est présente à raison de moins de 50 % en poids et en particulier moins de 20 % en poids de la composition.

### EMULSION

Au sens de la présente invention, on entend par « émulsion », un système de deux liquides non miscibles dont l'un est finement divisé en gouttelettes dans l'autre. La phase dispersée est encore appelée « phase interne ou discontinue ». La phase dispersante est aussi appelée « phase externe ou continue ». Les émulsions dans lesquelles la phase dispersée est lipophile, huile végétale ou minérale par exemple, et la phase dispersante hydrophile, par exemple l'eau, sont dites de type aqueux (H/E : huile-dans-eau). Les émulsions dans lesquelles la phase dispersée est hydrophile et la phase dispersante lipophile sont dites de type huileux (E/H : eau-dans-huile). Il existe également des émulsions dites multiples, par exemple E/H/E : eau-dans-huile-dans-eau.

Au sens de l'invention, les émulsions contiennent une phase lipophile et une phase hydrophile, cette dernière n'étant pas systématiquement de l'eau.

En particulier, la composition sous forme d'émulsion peut être translucide ou transparente et/ou peut être susceptible de donner un dépôt translucide ou transparent.

Selon un mode de réalisation, la composition sous forme d'émulsion contient au moins un agent émulsionnant et éventuellement au moins un agent co-émulsionnant en une proportion inférieure à 30 %, de préférence inférieure à 20 %, de préférence inférieure à 10%.

Selon un autre mode de réalisation, le composition contient au moins un agent émulsionnant et éventuellement au moins un agent co-émulsionnant en une proportion allant de 0,2 à 30 % en poids, de préférence de 0,3 à 20 % en poids et mieux de 0,5 à 15 % en poids par rapport au poids total de la composition.

De manière plus particulière, la composition contient moins de 0,5 % en poids par rapport au poids total de la composition d'agents émulsionnants et d'agents co-émulsionnants.

En particulier, la composition de l'invention sous forme d'une émulsion est avantageusement exempte de tout composé émulsionnant permettant de la stabiliser à l'exception du phyllosilicate exfolié conforme à l'invention.

L'homme du métier sait déterminer aisément, pour une composition comprenant une phase aqueuse et une phase grasse déterminées, dans un rapport déterminé, le type d'émulsionnant et éventuellement de co-émulsionnant de l'art antérieur ainsi que leurs proportions respectives permettant d'obtenir une émulsion stable.

Par émulsion stable, on entend une émulsion qui, lorsqu'elle est placée dans un récipient transparent, lui-même placé dans une étuve à 45 °C pendant deux mois, ne déphase pas (ou n'exsude pas) au bout de cette période. Le déphasage (ou exsudation) est détecté à l'oeil nu dès la sortie du récipient de l'étuve à travers ses parois transparentes.

La composition peut notamment se présenter sous forme d'émulsions comportant une phase aqueuse et une phase huileuse dispersées l'une dans l'autre, par exemple sous forme d'émulsions eau-dans-huile (E/H) ou huile-dans-eau (H/E) ou multiple (E/H/E ou H/E/H), choisies notamment parmi les émulsions habituelles ou les émulsions particulières comme par exemple parmi:
- les émulsions H/E à base de globules huileux pourvus d'un enrobage cristal liquide lamellaire, telles que décrites dans les documents EP-A-641557 et EP-A-705593 ;
- Les émulsions H/E sans tensioactif, stabilisées par des polymères anioniques hydrodispersibles, telles que celles décrites dans le document EP-A-864320 ;
- Les émulsions H/E à base de polymères dérivés d'acide 2-acrylamido 2-méthylpropane sulfonique (Polymère AMPS), telles que décrites dans le document EP-A-815844 ;
- Les émulsions H/E stabilisées par des polymères d'AMPS hydrophobes, telles décrites dans les documents EP-A-1,069,142, WO-A-2002/43689, WO-A-2002/44231, WO-A-2002/44271, WO-A-2002/44270, WO-A-2002/43686, WO-A-2002/44267, WO-A-2002/43688, WO-A-2002/43677, WO-A-2002/43687, WO-A-2002/44230 ;
- Les émulsions fluides à base de polymères thermo associatifs, telles que décrites dans les documents EP-A-1,355,990, EP-A-1,355,625, EP-A-1,307,501, EP-A-1,363,964 ;
- Les émulsions H/E obtenues par la méthode PIT (émulsion obtenue par inversion de phase, PIT : Phase Inversion Temperature), telles que décrites dans les documents WO-A-89/11907, DE-A-4318171, et EP-A-815846 ;
- Les nanoémulsions telles que celles décrites dans les demandes EP-A-728460, EP-A-780114, EP-A-780115, EP-A-879589, EP-A-1,010,413, EP-A-1,010,414, EP-A-1,010,415, EP-A-1,010,416, EP-A-1,013,338, EP-A-1,016,453, EP-A-1,018,363, EP-A-1,020,219, EP-A-1,025,898, EP-A-1,120,102, EP-A-1,120,101, EP-A-1,160,005, EP-A-1,172,077 et EP-A-1,353,629.

La proportion de la phase huileuse de l'émulsion peut varier de 1 à 80 % en poids, et de préférence de 1 à 50 % en poids par rapport au poids total de la composition. Les huiles, et les agents émulsionnants et co-émulsionnants éventuellement présents, utilisés dans la composition sous forme d'émulsion, sont choisis parmi ceux généralement utilisés dans le domaine cosmétique ou dermatologique. L'agent émulsionnant et l'agent co-émulsionnant, quand ils sont présents, sont généralement en proportion variant de 0,2 à 30 % en poids, de préférence de 0,3 à 20 % en poids et mieux de 0,5 à 15 % en poids par rapport au poids total de la composition. L'émulsion peut, en outre, contenir des vésicules lipidiques.

L'agent émulsionnant est choisi parmi les agents émulsionnants amphotères, anioniques, cationiques ou non ioniques, utilisés seuls ou en mélange. Les agents émulsionnants sont choisis de manière appropriée, suivant la phase continue de l'émulsion à obtenir (E/H ou H/E). Quand l'émulsion est multiple, elle comporte généralement un agent émulsionnant dans l'émulsion primaire et un émulsionnant dans la phase externe dans laquelle est introduite l'émulsion primaire.

Comme agents émulsionnants utilisables pour la préparation des émulsions E/H, on peut également citer les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme agents émulsionnants utilisables pour la préparation des émulsions H/E, on peut citer par exemple les agents émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ; les éthers d'alcools gras oxyalkylénés (oxyéthylénés et/ou oxypropylénés) ; les esters de sucres, alcoxylé ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces agents émulsionnants comme par exemple le mélange de stéarate de glycéryle et de stéarate de PEG-100, commercialisé sous la dénomination Arlacel 165 par la société Uniqema ; et les mélanges contenant ces émulsionnants, tels que le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate /cetearyl alcool /12-15 Pareth 7 / PPG 25 laureth 25).

On peut ajouter à ces agents émulsionnants, des agents co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations CARBOPOL 1342 et PEMULEN par la société Noveon ; ou les polymères en émulsion tels que celui commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate /isophtalate/1,4-cyclohexane-diméthanol (nom INCI : Diglycol/CHDM/Isophtalates/ SIP Copolymer) vendus sous les dénominations Eastman AQ polymer (AQ35S, AQ38S, AQ55S, AQ48 Ultra) par la société Eastman Chemical. On peut aussi préparer des émulsions sans émulsionnants, stabilisées par des particules siliconées ou des particules d'oxyde métallique tels que TiO2 ou autres.

### POLYMERES FILMOGENES

La composition selon l'invention peut comprendre, en outre, au moins un polymère filmogène sous réserve qu'il n'affecte pas la transparence ou translucidité requise selon l'invention. A ce titre, conviennent plus particulièrement les polymères filmogènes se présentant sous une forme solubilisée généralement dans la phase continue de ladite composition.

Toutefois, sous réserve de satisfaire à une taille de particules réduites notamment inférieure ou égale à 60 nm et à une polydispersité faible, les polymères filmogènes sous forme dispersés peuvent également convenir.

Selon la présente invention, on entend par « polymère filmogène », un polymère apte à former à lui seul ou en présence d'un agent auxiliaire de filmification, un film continu à l'oeil nu sur un support.

Pour préserver le caractère de transparence ou translucidité des compositions selon l'invention, l'emploi de polymères sous forme solubilisée est privilégié.

Il s'agit plus particulièrement de polymères liposolubles notamment dans le cas où la composition cosmétique comprend au moins une phase grasse liquide.

Ces polymères liposolubles peuvent être de toute nature chimique et englobent notamment :
a) les homopolymères et les copolymères liposolubles et amorphes d'oléfines, de cyclo-oléfines, de butadiene, d'isoprène, de styrène, d'éthers, d'esters ou d'amides vinyliques, d'esters ou d'amides d'acide (méth)acrylique comprenant un groupement alkyl en C₄₋₅₀ linéaire, ramifié ou cyclique, et de préférence amorphe.
   Comme copolymères liposolubles particuliers, on peut citer :
   i) les polymères greffés acrylique-silicone à squelette silicone, greffons acryliques ou à squelette acrylique, greffons silicones tels que le produit vendu sous la dénomination SA 70.5 par 3M et ceux décrits dans les brevets US 5 725 882, US 5 209 924, US 4 972 037, US 4 981 903, US 4 981 902, US 5 468 477, US 5 219 560 et EP 0 388 582.
   ii) les polymères liposolubles porteurs de groupes fluorés appartenant à l'une des classes décrites ci-dessus, en particulier ceux décrits dans le brevet US 5 948 393, les copolymères (méth)acrylate d'alkyle / (méth)acrylate de perfluoroalkyle décrits dans les brevets EP 0 815 836 et US 5 849 318.
   iii) les polymères ou copolymères résultant de la polymérisation ou de la copolymérisation d'un monomère éthylénique, comprenant un ou plusieurs motifs éthyléniques, de préférence conjugués (ou diènes). Comme polymères ou copolymères résultant de la polymérisation ou de la copolymérisation d'un monomère éthylénique, on peut utiliser les copolymères vinyliques, acryliques ou méthacryliques qui peuvent être des copolymères en blocs, tels que les copolymères di-blocs ou tri-blocs, ou même des copolymères multiblocs à formes variées. L'agent filmogène, comprenant au moins un motif éthylénique, peut comprendre, par exemple, un bloc styrène (S), un bloc alkylstyrène (AS), un bloc éthylène/butylène (EB), un bloc éthylène/propylène (EP), un bloc butadiène (B), un bloc isoprène (I), un bloc acrylate (A), un bloc méthacrylate (MA) ou une combinaison de ces blocs.
   iv) les copolymères de N-vinyl pyrrolidone et d'oléfines, oléfines dont le nombre d'atomes de carbone est > à 8 ex copolymère N-vinyl pyrrolidone/hexadécène, N-vinyl pyrrolidone/eicosène.
   v) les homo ou copolymères d'esters vinyliques liposolubles comme les polylaurates de vinyle, polystéarate de vinyle et leurs copolymères avec l'acétate de vinyle.
   vi) les homo ou copolymères d'esters ou d'amides(méth)acryliques. Les monomères esters méthacryliques résultent alors de l'estérification de l'acide (meth)acrylique avec un alcool ayant un nombre d'atomes de carbone > 4 et de préférence ≥ à 8 avec comme exemple (méth)acrylate 2-éthylhexyle, (méth)acrylate de n-octyle, (méth)acrylate de dodécyle, (méth)acrylate de stéaryle, (méth)acrylate de béhényle. Ces (méth)acrylates à longue chaîne peuvent être copolymérisés avec d'autres esters (méth)acryliques, esters vinyliques ou styrène.
b) les polycondensats liposolubles, amorphes, notamment ne comportant pas de groupes donneurs de liaisons hydrogène, en particulier les polyesters à chaînes latérales alkyles en C₄₋₅₀, les polyesters résultant de la condensation d'acides gras dimères, ou les polyesters comportant un segment silicone sous forme de séquence, greffon, ou groupe terminal, solides à température ambiante,
c) les polysaccharides liposolubles et amorphes comportant des chaînes latérales alkyles (éthers ou esters), en particulier les alkylcelluloses portant des radicaux alkyles linéaires ou ramifiés, saturés ou insaturés en C₁ à C₈, tels que l'éthylcellulose et le propylcellulose.

D'une façon générale, les polymères liposolubles filmogènes susceptibles d'être mis en oeuvre selon l'invention peuvent avoir un poids moléculaire compris entre 1.000 et 500.000, de préférence entre 2.000 et 250.000, et une température de transition vitreuse comprise entre -100°C et +300°C, notamment entre -50°C et +100°C, et en particulier entre -10°C et +90°C.

Dans le cas particulier des compositions cosmétiques comprenant au moins une phase organique non miscible à l'eau et notamment de type vernis à ongles, conviennent tout particulièrement à titre d'agent filmogène, les nitrocelluloses et/ou les esters de cellulose tels que les acétates de cellulose, les propionates de cellulose, les butyrates de cellulose, les acétopropionates de cellulose, les acétobutyrates de cellulose et leurs mélanges.

Les polymères dans la composition selon l'invention peuvent être également utilisés en une quantité variant de 0,01 % à 40 % par rapport au poids total de la composition, notamment de 1 à 20 %, tel que, par exemple, de 1 % à 10%.

Le polymère filmogène peut être associé à des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

### AGENT DE COLORATION

Comme précisé précédemment les compositions selon l'invention peuvent être colorées.

La composition selon l'invention peut notamment comprendre des agents colorants choisis parmi les colorants hydrosolubles, les colorants liposolubles, les pigments et les laques.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicétopyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en phyllosilicates intercalés exfoliés présente dans la composition cosmétique considérée.

Les nacres peuvent être présentes dans la composition à raison de 0,01 à 25 % en poids.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan III (CFTA : D & C Red 17), la lutéine, le vert de quinizarine (CFTA : D & C green 6), le pourpre d'alizurol SS (CTFA : D & C violet n° 2), les dérivés des caroténoïdes tels que le lycopène, le bétacarotène, la bixine ou la capsantéine, les dérivés de rocou et de fuschine, le DC orange 5, et le jaune quinoléine.

Les colorants hydrosolubles sont par exemple le bleu de méthylène et les extraits de plantes tels que le jus de betterave, les extraits *de sorgho, de Pterocarpus soyauxil, de Monascus, de Lawsonia inermis, de Mercurialis perenis, d'Helianthus aanus, d'Impatiens balsamina, de Curcuma longa, de Phytolacca décandra, de Solidago aureus, de Juglans regia, d'Iris germanica, d'Alkanna tinctoria, de Chrozophora tinctoria, d'Isatis tinctoria.*

Les laques utilisables dans les compositions de la présente invention sont par exemple les laques à base de carmin de cochenille ou à base de sels de calcium, de baryum, d'aluminium, de strontium ou de zirconium, de colorants acides.

La quantité d'agent colorant est déterminante pour la présente invention. En dessous d'une certaine quantité d'agent colorant, la composition donnera lieu à un dépôt suffisamment transparent ou translucide pour préserver l'aspect naturel de la peau, des lèvres ou des phanères, mais elle ne permettra pas d'apporter une coloration visible à l'oeil nu.

Au contraire, pour une proportion d'agent colorant trop importante, la couleur du dépôt de maquillage sera certes visible mais la transparence ou la translucidité de celui-ci sera insuffisante pour préserver l'aspect naturel de la peau. Par ailleurs, des compositions cosmétiques contenant une proportion trop importante d'agent colorant seront insuffisamment transparentes ou translucides dans la masse.

En conséquence, cette quantité est ajustée en prenant compte du test décrit précédemment dans le chapitre transparence-translucidité.

Les compositions cosmétiques selon la présente invention contiennent généralement de 0,05 % à 20 % en poids et de préférence de 0,1 à 15 %, et en particulier de 0,5 à 10 % en poids d'agent colorant.

Dans un mode de réalisation particulier de la présente invention, la composition cosmétique colorée contient au moins un colorant hydrosoluble ou liposoluble.

Dans un autre mode de réalisation particulièrement intéressant de l'invention, la composition contient, en tant qu'agent colorant uniquement un ou plusieurs colorants solubles dans la phase grasse liquide, et est exempte d'agents colorants insolubles de type pigments ou laques.

De telles compositions contenant uniquement des colorants solubles ont en effet un bon pouvoir colorant associé à d'excellentes propriétés de transparence dues à l'absence de diffusion de la lumière par des particules insolubles.

La composition selon l'invention peut, de plus, comprendre tous les ingrédients classiquement utilisés dans les domaines concernés et plus spécialement dans le domaine cosmétique et dermatologique et/ou soin des ongles sous réserve bien sûr qu'ils n'affectent pas la translucidité ou la transparence requise selon l'invention.

Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges.

Ainsi, lorsque les compositions selon l'invention sont plus particulièrement destinées au soin des ongles naturels, elles peuvent notamment incorporer, à titre d'actifs, des agents durcissants pour matières kératiniques et/ou des actifs pour traiter des affections diverses localisées au niveau de l'ongle, comme par exemple l'onichomycose.

La composition selon l'invention, peut également contenir des ingrédients couramment utilisés dans le domaine cosmétique et/ou soin des ongles.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie.

Selon un mode préféré de l'invention, on utilisera pour les formulations cosmétiques des argiles, de préférence montmorillonites, pré-intercalées que l'on va exfolier :
a) soit dans un pré-gel contenant au moins un des composants de la phase grasse liquide finale. Il peut s'agir par exemple d'une une huile hydrocarbonée polaire ou non polaire, une silicone volatile ou non volatile,
b) soit directement dans la formulation, c'est-à-dire en présence des autres solvants et/ou huiles et/ou polymères et/ou pigments et charges.

Que ce soit selon le mode a) ou le mode b), l'exfoliation des phyllosilicates intercalés est réalisée par tous moyens susceptibles de conduire à une délamination d'au moins environ 80 % en poids dudit phyllosilicate. En l'occurrence, la vitesse de cisaillement requise pour réaliser ce type d'exfoliation peut nécessiter une vitesse de cisaillement d'au moins 10s⁻¹, voire plus. En l'occurrence, la limite supérieure de cette vitesse de cisaillement n'est pas critique.

Généralement, cette vitesse de cisaillement varie d'environ 10s⁻¹ à environ 20 000s⁻¹ et plus particulièrement d'environ 100s⁻¹ à environ 10 000s⁻¹. Dans certains cas, il peut être avantageux d'associer à ce cisaillement un chauffage et/ou une augmentation de la pression.

En ce qui concerne plus particulièrement le cisaillement en soit, il peut être assuré à l'aide de différents dispositifs conventionnels. Ce cisaillement peut notamment être réalisé à l'aide de moyens mécaniques, par choc thermique, altération de pression ou par ultrasons. Le choix du mode de cisaillement relève de la connaissance de l'homme de l'art.

Conviennent tout particulièrement à l'invention, les cisaillements obtenus par des modes mécaniques, comme notamment les agitateurs, homogénéisateurs ou disperseurs de type MORITZ^{®}, ULTRATURRAX^{®}, le mixeur de type BANBURY^{®}, les mélangeurs de type BRABENDER^{®} et les extrudeurs, notamment de type KNEADER^{®}.

Dans le cas particulier d'un cisaillement mécanique, notamment dans une extrudeuse, la température du milieu à exfolier, la longueur de l'extrudeuse, le temps de résidence de ce milieu dans l'extrudeuse et le type d'extrudeuse retenue, à savoir simple vis/double vis ..., sont autant de variables susceptibles de contrôler la force de cisaillement à appliquer pour l'exfoliation.

Une exfoliation est jugée suffisante lorsqu'elle procure au moins 80 % en poids, notamment au moins 85 %, en particulier au moins environ 90 % en poids, voire environ 95 % en poids de phyllosilicate intercalé et exfolié.

Les phyllosilicates exfoliés ainsi formés possèdent avantageusement une épaisseur variant de l'épaisseur de couches individuelles à l'épaisseur de une à cinq couches associées.

La composition peut se présenter sous diverses formes, en fonction de sa destination. La composition cosmétique peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, d'une émulsion multiple, d'une dispersion d'huile dans de l'eau grâce à des vésicules situés à l'interface huile/eau, d'une émulsion directe ou inverse.

La composition peut se présenter sous forme de produit coulé, en coupelle ou sous forme de stick dans le cas d'un rouge à lèvres, d'un produit de soin des lèvres, ou d'un fond de teint.

La composition peut encore se présenter sous diverses autres formes, par exemple d'un liquide plus ou moins visqueux, d'un gel ou d'une pâte.

La composition cosmétique peut constituer, entre autres, un rouge à lèvres, un baume à lèvres, un gloss liquide, une pâte de rouge à lèvres, un fard à joues, un vernis à ongles, un crayon à lèvres, un fond de teint solide, notamment coulé, ou fluide, de produits « correcteurs » ou « embellisseurs » de teint, un produit pour le soin et/ou le maquillage des ongles naturels ou synthétiques, un produit anti-cernes ou de contour des yeux, un eye-liner, un mascara, une ombre ou un fard à paupières, un produit de maquillage du corps ou des cheveux ou encore un produit solaire ou de coloration de la peau.

En particulier, il s'agit d'un vernis à ongles.

Les exemples figurant ci-après sont présentés à titre illustratif et non limitatif du domaine de l'invention.

### Exemple 1 et exemple comparatif: Préparation de vernis à ongles

On prépare un vernis selon l'invention en formulant des phyllosilicates constitués de montmorillonite modifiée par une amine grasse d'origine suif commercialisé sous la référence Nanomer I.34TCN par Nanocor.

On prépare également selon le même procédé un vernis de l'art antérieur de même composition en remplaçant poids pour poids les phyllosilicates, par de l'hectorite modifiée par du chlorure de stéaryl diméthyl benzyl ammonium commercialisée sous la référence Bentone^{®} 27V par Elementis.
a) On réalise un gel nitrocellulosique thixotrope en mélangeant à 25°C sous une agitation appropriée
   - 13 g de nitrocellulose (agent filmogène)
   - 7 g de phyllosilicate intercalé ou d'hectorite
   - 0,3 g d'acide citrique (agent gonflant)
   - 8 g d'alcool isopropylique (solvant volatil)
   - qsp 100 g d'acétate de butyle
   100 parties du gel ainsi obtenu sont mélangées à 25°C à 450 parties d'une base incolore non thixotrope de composition comme suit:
   - 8 g de plastifiant
   - 24 g d'un mélange de nitrocellulose et d'un agent co-filmogène
   - 5 g d'alcool isopropylique
   - acétate d'éthyle/acétate de butyle 50/50 q.s.p 100 g

On obtient ainsi un vernis à ongle dont la composition est la suivante (pourcentages en poids) :
- agent filmogène (nitrocellulose, résine ...) 22 %
- plastifiant 6,5 %
- alcool isopropylique 5,5 %
- phyllosilicate ou hectorite 1,3%
- acide citrique 0,05 %
- acétate de butyle 39,2 %
- acétate d'éthyle 26,1%

La brillance de cette composition est mesurée à l'aide d'un brillancemètre, de manière conventionnelle par la méthode suivante.

Sur une carte de contraste de marque LENETA et de référence FORM 1A PENOPAC, on étale une couche d'environ 300 µm d'épaisseur de la composition à l'aide d'un étaleur automatique. La couche recouvre au moins le fond blanc de la carte. On laisse le dépôt sécher. On procède à la mesure de la brillance à 20° et à 60° sur le fond blanc à l'aide d'un brillancemètre de marque BYK GARDNER et de référence micro TRI-GLOSS. On obtient une valeur de la brillance moyenne comprise entre 0 et 100.

Les valeurs mesurées sont indiquées ci-après pour chacune des compositions testées.

| | Vernis transparent préparé avec la bentone B27 V commercialisée par Elementis | Vernis transparent préparé avec le phyllosilicate Nanomer I.34TCN commercialisé par Nanocor |
|---|---|---|
| Brillance à 20°/à 60° | 53/85 | 63/86 |

### Exemple 2 et exemple comparatif: Préparation d'un vernis à ongles

Dans un malaxeur bi-vis, on malaxe :
- 20 g d'hectorite ou de phyllosilicate tels que définis en exemple 1,
- 30 g d'acétobutyrate de cellulose d'Eastman Chemical CAB 3810.5,
- 50 g de n-éthyl-o,p-toluène-sulfonamide de PAN-AMERICANA (référence Resimpol 8).

5 parties du gel obtenu ci-dessus sont mélangées à 95 parties du gel nitrocellulosique thixotrope obtenu en exemple 1 *a).*

La brillance de cette composition est mesurée à l'aide d'un brillancemètre, de manière conventionnelle par la méthode suivante.

Sur une carte de contraste de marque LENETA et de référence FORM 1A PENOPAC, on étale une couche d'environ 300 µm d'épaisseur de la composition à l'aide d'un étaleur automatique. La couche recouvre au moins le fond blanc de la carte. On procède de suite à la mesure de la brillance à 20° et à 60° sur le fond blanc à l'aide d'un brillancemètre de marque BYK GARDNER et de référence micro TRI-GLOSS. On obtient une valeur de la brillance moyenne comprise entre 0 et 100.

Les valeurs mesurées sont indiquées ci-après pour chacune des compositions testées.

| | Vernis transparent préparé avec la bentone B27 V commercialisée par Elementis | Vernis transparent préparé avec le phyllosilicate Nanomer I.34TCN commercialisé par Nanocor |
|---|---|---|
| Brillance 20°/60° | 54/79 | 71/87 |

## Revendications

1. Composition cosmétique de vernis à ongles comprenant au moins :
- une phase grasse liquide, ou
- une phase organique non miscible à l'eau, ladite phase organique comprenant un ou plusieurs solvants organiques dont la miscibilité dans l'eau à 25 °C est inférieure ou égale à 50 % en poids,
et ladite phase étant structurée par au moins une quantité efficace de phyllosilicate exfolié dérivant d'au moins un phyllosilicate intercalé.

2. Composition selon la revendication 1, **caractérisée en ce que** les phyllosilicates exfoliés possèdent un facteur de forme variant de 50 à 2000, notamment de 75 à 1500 et en particulier de 200 à 1000.

3. Composition selon la revendication 1 ou 2, **caractérisée en ce qu'**au moins 50 %, notamment 70 %, en particulier 90 %, voire 95 % de phyllosilicates exfoliés sont formées de moins de dix feuillets, notamment moins de 5 feuillets, en particulier moins de 3 feuillets, voire d'un seul feuillet de phyllosilicate.

4. Composition selon l'une quelconque des revendications précédentes, **caractérisée en ce qu'**elle possède une teneur en phyllosilicates exfoliés variant de 0,05 à 30 % en poids, notamment de 0,1 à 20 % en poids, et en particulier de 0,5 à 10 % en poids par rapport au poids total de la composition.

5. Composition selon l'une quelconque des revendications précédentes, **caractérisée en ce que** les phyllosilicates exfoliés dérivent de l'intercalation d'argiles minérales de type smectite.

6. Composition selon l'une quelconque des revendications précédentes, **caractérisée en ce que** les smectites sont choisis parmi les montmorillonites, nontronites, beidellites, volkonskoïtes, hectorites, saponites, sauconites, sobockites, stevensites, svinfordites, vermiculites et leurs mélanges.

7. Composition selon l'une quelconque des revendications précédentes, **caractérisée en ce que** les phyllosilicates exfoliés possèdent au moins 15 % en poids, notamment au moins 20 % en poids et plus particulièrement au moins 30 % en poids d'agent intercalant par rapport au poids de phyllosilicate sec comprenant moins de 5 % d'eau.

8. Composition selon l'une quelconque des revendications précédentes, **caractérisée en ce que** l'agent intercalant est choisi parmi :
a) les oniums possédant au moins une chaîne hydrocarbonée en C₁ à C₅₀,
b) les composés organiques, de nature polymérique ou non, possédant au moins un cycle aromatique ou au moins un groupe polaire choisi parmi les groupes carbonyle, hydroxyle, polyol, acide carboxylique, aldéhyde, cétone, amine, amide linéaire ou cyclique, ester, lactone, éther, sulfate, sulfonate, sulfinate, sulfanate, phosphate, phosphonate et phosphinate.

9. Composition selon la revendication 8, **caractérisée en ce que** le cation onium est choisi parmi les ammoniums primaires, secondaires, tertiaires ou quaternaires possédant au moins une chaîne alkyle en C₄-C₁₀.

10. Composition selon la revendication 8 ou 9, **caractérisée en ce que** le composé organique est un composé non polymérique possédant un groupe polaire et au moins une chaîne hydrophobe, notamment une chaîne alkyle en C₄ à C₅₀, alkylène en C₄ à C₅₀ et/ou alkylaryle en C₄ à C₅₀.

11. Composition selon la revendication 10, **caractérisée en ce que** le composé organique non polymérique est choisi parmi les alkylpyrrolidones avec une chaîne alkyle en C₄ à C₅₀, en particulier en C₈ à C₃₀.

12. Composition selon la revendication 8, **caractérisée en ce que** le composé organique est un oligomère ou polymère synthétique possédant au moins un noyau aromatique ou un groupe polaire et notamment choisi parmi les dérivés polyvinylpyrrolidones (PVP), les dérivés polyvinylalcools (PVA), les dérivés polyacryliques sous leur forme polymérique et copolymérique, les dérivés acides polyméthacryliques (PMAA), les dérivés polyvinyloxazolidones (PVO) et polyvinylméthyloxazolidones (PVMO), les dérivés polyvinyloxazolines.

13. Composition selon l'une quelconque des revendications précédentes, **caractérisée en ce qu'**elle se présente sous la forme d'une émulsion.

14. Composition selon la revendication précédente, **caractérisée en ce que** l'émulsion est de type directe ou inverse.

15. Composition selon l'une des revendications 13 ou 14, **caractérisée en ce que** l'émulsion est de type eau-dans-huile ou huile-dans-eau ou multiple.

16. Composition selon l'une quelconque des revendications 13 à 15, **caractérisée en ce qu'**elle comprend un agent émulsionnant, et éventuellement un agent co-émulsionnant, en proportion inférieure à 30 % en poids, et en particulier inférieure à 20 % en poids, et plus particulièrement inférieure à 10 %.

17. Composition selon l'une quelconque des revendications 13 à 15, **caractérisée en ce qu'**elle est dépourvue d'agents émulsionnants et d'agents co-émulsionnants.

18. Composition selon l'une quelconque des revendications précédentes, **caractérisée en ce qu'**elle comprend une phase grasse continue.

19. Composition selon l'une quelconque des revendications précédentes, **caractérisée en ce qu'**elle est anhydre.

20. Composition selon l'une quelconque des revendications précédentes, **caractérisée en ce que** ladite phase grasse contient en outre au moins un corps gras liquide à température ambiante et à pression atmosphérique et le cas échéant au moins un corps gras solide à température ambiante et à pression atmosphérique.

21. Composition selon la revendication 20, **caractérisée en ce que** ledit corps gras liquide à température ambiante et à pression atmosphérique comprend au moins une huile non volatile.

22. Composition selon la revendication 21, **caractérisée en ce que** l'huile non volatile est choisie parmi les huiles hydrocarbonées d'origine animale, les huiles hydrocarbonées végétales, les hydrocarbures linéaires ou ramifiés d'origine minérale ou synthétique, les éthers de synthèse ayant de 10 à 40 atomes de carbone; les esters de synthèse, les esters de polyol, les alcools gras liquides à température ambiante ayant de 12 à 26 atomes de carbone, les acides gras supérieurs, les huiles siliconées de type polyméthylsiloxanes (PDMS) et leurs mélanges.

23. Composition selon l'une quelconque des revendications 20 à 22, **caractérisée en ce que** le corps gras liquide comprend au moins une huile volatile choisie parmi les huiles hydrocarbonées ayant de 8 à 16 atomes de carbone et les huiles de silicones volatiles.

24. Composition selon l'une quelconque des revendications précédentes, **caractérisée en ce qu'**elle comprend une phase huileuse en proportion variant de 1 à 80 %, et en particulier de 1 à 50 %, par rapport au poids total de la composition.

25. Composition selon l'une quelconque des revendications précédentes **caractérisée en ce que** ladite phase organique non miscible à l'eau comprend un ou plusieurs solvants organiques dont la miscibilité dans l'eau est inférieure ou égale à 50% en poids à 25 °C.

26. Composition selon la revendication précédente **caractérisée en ce que** ledit solvant est choisi parmi les esters à chaînes courtes ayant de 4 à 8 atomes de carbone au total tels que l'acétate d'éthyle, les acétates de propyle, de butyle, d'aryle, l'acétate d'isopentyle et leurs mélanges.

27. Composition selon l'une quelconque des revendications précédentes, **caractérisée en ce qu'**elle comprend au moins un agent colorant.

28. Composition selon la revendication précédente, **caractérisée en ce que** l'agent colorant est choisi parmi les colorants hydrosolubles, les colorants liposolubles, les pigments et les laques.

29. Composition selon la revendication 27 ou 28, **caractérisée en ce qu'**elle comprend de 0,05 à 20 % et notamment de 0,1 à 15 % et en particulier de 0,5 à 10 % en poids d'agent colorant.

30. Composition selon l'une quelconque des revendications précédentes, **caractérisée en ce qu'**elle est translucide ou transparente et/ou susceptible de donner un dépôt translucide ou transparent.

31. Utilisation de phyllosilicates exfoliés telles que définies selon l'une quelconque des revendications 1 à 12 à titre d'agent pour texturer la phase grasse liquide ou la phase organique d'une composition de vernis à ongles, ladite phase étant non miscible à l'eau et comprenant un ou plusieurs solvants organiques dont la miscibilité dans l'eau à 25 °C est inférieure ou égale à 50 % en poids.

32. Utilisation selon la revendication précédente, **caractérisée en ce que** la composition est une émulsion.

33. Utilisation selon la revendication 31 ou 32, **caractérisée en ce que** la composition est transparente ou translucide et/ou susceptible de donner un dépôt transparent ou translucide.

34. Procédé de maquillage des phanères comprenant l'application sur les phanères d'au moins une couche d'une composition telle que définie selon l'une quelconque des revendications 1 à 30.

35. Support synthétique maquillé sur lequel est présent en tout ou partie de sa surface une composition telle que définie selon l'une quelconque des revendications 1 à 30.

## Claims

1. Cosmetic composition of nail varnish comprising at least
- one liquid fatty phase, or
- one water-immiscible organic phase, the said organic phase comprising one or more organic solvents having a miscibility in water at 25°C which is less than or equal to 50% by weight and the said phase being structured by at least an effective amount of exfoliated phyllosilicate deriving from at least one intercalated phyllosilicate.

2. Composition according to Claim 1, **characterized in that** the exfoliated phyllosilicates have an aspect ratio varying from 50 to 2000, in particular from 75 to 1500 and especially from 200 to 1000.

3. Composition according to Claim 1 or 2, **characterized in that** at least 50%, in particular 70%, especially 90%, indeed even 95% of exfoliated phyllosilicates are formed of less than ten sheets, in particular less than 5 sheets, especially less than 3 sheets, indeed even of a single sheet of phyllosilicate.

4. Composition according to any one of the preceding claims, **characterized in that** it has a content of exfoliated phyllosilicates varying from 0.05 to 30% by weight, in particular from 0.1 to 20% by weight and especially from 0.5 to 10% by weight with respect to the total weight of the composition.

5. Composition according to any one of the preceding claims, **characterized in that** the exfoliated phyllosilicates derive from the intercalation of mineral clays of smectite type.

6. Composition according to any one of the preceding claims, **characterized in that** the smectites are chosen from montmorillonites, nontronites, beidellites, volkonskoites, hectorites, saponites, sauconites, sobockites, stevensites, svinfordites, vermiculites and their mixtures.

7. Composition according to any one of the preceding claims, **characterized in that** the exfoliated phyllosilicates have at least 15% by weight, in particular at least 20% by weight and more particularly at least 30% by weight of intercalating agent with respect to the weight of dry phyllosilicate comprising less than 5% of water.

8. Composition according to any one of the preceding claims, **characterized in that** the intercalating agent is chosen from:
a) oniums having at least one C₁ to C₅₀ hydrocarbonaceous chain,
b) organic compounds of polymeric or nonpolymeric nature having at least one aromatic ring or at least one polar group chosen from the carbonyl, hydroxyl, polyol, carboxylic acid, aldehyde, ketone, amine, linear or cyclic amide, ester, lactone, ether sulfate, sulfonate, sulfinate, sulfanate, phosphate, phosphonate, and phosphinate groups.

9. Composition according to Claim 8, **characterized in that** the onium cation is chosen from primary, secondary, tertiary or quaternary ammoniums having at least one C₄-C₁₀ alkyl chain.

10. Composition according to Claims 8 or 9, **characterized in that** the organic compound is a nonpolymeric compound having a polar group and at least one hydrophobic chain, in particular a C₄ to C₅₀ alkyl, C₄ to C₅₀ alkylene and/or C₄ to C₅₀ alkylaryl chain.

11. Composition according to Claim 10, **characterized in that** the nonpolymeric organic compound is chosen from alkylpyrrolidones with a C₄ to C₅₀, in particular C₈ to C₃₀, alkyl chain.

12. Composition according to Claim 8, **characterized in that** the organic compound is a synthetic oligomer or polymer having at least one aromatic ring or one polar group and chosen in particular from polyvinylpyrrolidone (PVP) derivatives, polyvinyl alcohol (PVA) derivatives, polyacrylic derivatives in their polymeric and copolymeric form, polymethacrylic acid (PMAA) derivatives, polyvinyloxazolidone (PVO) and polyvinylmethyloxazolidone (PVMO) derivatives, polyvinyloxazoline derivatives.

13. Composition according to any one of the preceding claims, **characterized in that** it is in the form of an emulsion.

14. Composition according to the preceding claim, **characterized in that** said emulsion is of direct or inverse type.

15. Composition according to claims 13 or 14, **characterized in that** said emulsion is in form of water-in-oil or oil-in-water or multiple emulsion.

16. Composition according to any one of the claims 13 to 15, **characterized in that** said emulsion comprises an emulsifying agent, and if appropriate a co-emulsifying agent, in an amount less than 30%, in particular, less than 20%, in particular less than 10% by weight.

17. Composition according to any one of the claims 13 to 15, **characterized in that** said emulsion is free of any emulsifying and co-emulsifying agent.

18. Composition according to any one of the preceding claims, **characterized in that** it comprises a continuous fatty phase.

19. Composition according to any one of the preceding claims, **characterized in that** it is anhydrous.

20. Composition according to any one of the preceding claims, **characterized in that** the said fatty phase comprises in addition at least one fatty substance which is liquid at ambient temperature and at atmospheric pressure and, if appropriate, at least one fatty substance which is solid at ambient temperature and at atmospheric pressure.

21. Composition according to Claim 20, **characterized in that** the said fatty substance which is liquid at ambient temperature and at atmospheric pressure comprises at least one nonvolatile oil.

22. Composition according to Claim 21, **characterized in that** the nonvolatile oil is chosen from hydrocarbonaceous oils of animal origin; vegetable hydrocarbonaceous oils; linear or branched hydrocarbons of mineral or synthetic origin; synthetic ethers having from 10 to 40 carbon atoms; synthetic esters, polyol esters; fatty alcohols which are liquid at ambient temperature having from 12 to 26 carbon atoms; higher fatty acids; silicone oils of polymethylsiloxane (PDMS) type, and their mixtures.

23. Composition according to any one of Claims 20 to 22, **characterized in that** the liquid fatty substance comprises at least one volatile oil chosen from hydrocarbonaceous oils having from 8 to 16 carbon atoms and volatile silicone oils.

24. Composition according to any one of the preceding claims, **characterized in that** it comprises an oily phase in an amount ranging from 1 to 80% by weight, in particular from 1 to 50% by weight relative to the total weight of the composition.

25. Composition according to any one of the preceding claims, **characterized in that** the said water-immiscible organic phase comprises one or more organic solvents having a miscibility in water of less than or equal to 50% by weight, at 25°C.

26. Composition according to the preceding claim, **characterized in that** the said solvent is chosen from short-chain esters having from 4 to 8 carbon atoms in total, such as ethyl acetate, propyl acetate, butyl and aryl acetates, isopentyl acetate and their mixtures.

27. Composition according to any one of the preceding claims, **characterized in that** it comprises at least one colouring agent.

28. Composition according to the preceding claim, **characterized in that** the colouring agent is chosen from water-soluble dyes, fat-soluble dyes, pigments and lakes.

29. Composition according to Claim 27 or 28, **characterized in that** it comprises from 0.05 to 20% and in particular from 0.1 to 15%, and more particularly from 0.5 to 10% by weight of colouring agent.

30. Composition according to any one of the preceding claims, **characterized** it is transparent or translucent, and/or is capable of giving a transparent or translucent coat.

31. Use of exfoliated phyllosilicates according to any one of Claims 1 to 12 as agent for texturizing the liquid fatty phase or the organic phase of a nail varnish composition, the said phase being water-immiscible and comprising one or more organic solvents having a miscibility in water at 25°C less than or equal to 50% by weight.

32. Use according to the preceding claim, **characterized in that** the composition is in the form of an emulsion.

33. Use according to the claims 31 or 32, **characterized in that** the composition is transparent or translucent and/or is capable of giving a transparent or translucent coat.

34. Process for making up the exoskeleton comprising the application, to the exoskeleton, of at least one layer of a composition as defined according to any one of Claims 1 to 30.

35. Made-up synthetic substrate on which is present, over all or part of its surface, a composition as defined according to any one of Claims 1 to 30.

## Patentansprüche

1. Kosmetische Nagellack-Zusammensetzung, die mindestens folgendes umfasst:
- eine flüssige Fettphase, oder
- eine nicht mit Wasser mischbare organische Phase, wobei die organische Phase ein oder mehrere organische Lösungsmittel umfasst, deren Mischbarkeit in Wasser bei 25 °C kleiner oder gleich 50 Gew.-% ist,a
und die Phase durch mindestens eine wirksame Menge von exfoliertem Phyllosilikat strukturiert ist, das sich von mindestens einem interkalierten Phyllosilikat ableitet.

2. Zusammensetzung nach Anspruch 1, **dadurch gekennzeichnet, dass** die exfolierten Phyllosilikate einen Formfaktor aufweisen, der von 50 bis 2000, insbesondere von 75 bis 1500 und speziell von 200 bis 1000 variiert.

3. Zusammensetzung nach Anspruch 1 oder 2, **dadurch gekennzeichnet, dass** mindestens 50 %, insbesondere 70 %, speziell 90 %, und sogar 95 % von exfolierten Phyllosilikaten aus weniger als 10 Schichten, insbesondere aus weniger als 5 Schichten, speziell weniger als 3 Schichten, und sogar aus einer Phyllosilikatschicht gebildet sind.

4. Zusammensetzung nach einem der vorhergehenden Ansprüche, **dadurch gekennzeichnet, dass** sie einen Gehalt an exfolierten Phyllosilikaten besitzt, der von 0,05 bis 30 Gew.-%, insbesondere von 0,1 bis 20 Gew.-% und speziell von 0,5 bis 10 Gew.-% variiert, bezogen auf das Gesamtgewicht der Zusammensetzung.

5. Zusammensetzung nach einem der vorhergehenden Ansprüche, **dadurch gekennzeichnet, dass** die exfolierten Phyllosilikate sich von der Interkalation von mineralischen Tonen vom Typ eines Smektits ableiten.

6. Zusammensetzung nach einem der vorhergehenden Ansprüche, **dadurch gekennzeichnet, dass** die Smektite ausgewählt sind aus Montmorilloniten, Nontroniten, Beidelliten, Volkonskoiten, Hektoriten, Saponiten, Sauconiten, Sobockiten, Stevensiten, Svinforditen, Vermikuliten und ihren Gemischen.

7. Zusammensetzung nach einem der vorhergehenden Ansprüche, **dadurch gekennzeichnet, dass** die exfolierten Phyllosilikate, bezogen auf das Gewicht von trockenem Phyllosilikat, das weniger als 5 Gew.-% Wasser einschließt, mindestens 15 Gew.-%, insbesondere mindestens 20 Gew.-% und spezieller mindestens 30 Gew.-% an Interkalationsmittel aufweisen.

8. Zusammensetzung nach einem der vorhergehenden Ansprüche, **dadurch gekennzeichnet, dass** das Interkalationsmittel ausgewählt ist aus:
a) Onium-Ionen, die die mindestens eine C₁-C₅₀-Kohlenwasserstoffkette aufweisen,
b) organischen Verbindungen polymerer oder nicht polymerer Natur, die mindestens einen aromatischen Cyclus oder mindestens eine polare Gruppe aufweisen, ausgewählt aus Carbonyl-, Hydroxyl-, Polyol-, Carbonsäure-, Aldehyd-, Keton-, Amin-, linearen oder cyclischen Amid-, Ester-, Lakton-, Ether-, Sulfat-, Sulfonat-, Sulfinat-, Sulfanat-, Phosphat-, Phosphonat- und Phosphinatgruppen.

9. Zusammensetzung nach Anspruch 8, **dadurch gekennzeichnet, dass** das Onium-Kation aus primärem, sekundärem, tertiärem oder quaternärem Ammonium ausgewählt ist, das mindestens eine C₄-C₁₀-Alkylkette besitzt.

10. Zusammensetzung nach Anspruch 8 oder 9, **dadurch gekennzeichnet, dass** die organische Verbindung eine nicht polymere Verbindung ist, die eine polare Gruppe und mindestens ein hydrophobe Kette, insbesondere eine C₄-C₅₀-Alkylkette, C₄-C₅₀-Alkylenkette und/oder C₄-C₅₀-Alkylarylkette besitzt.

11. Zusammensetzung nach Anspruch 10, **dadurch gekennzeichnet, dass** die organische nicht polymere Verbindung aus Alkylpyrrolidonen mit einer C₄-C₅₀-, insbesondere einer C₈-C₃₀-Alkylkette ausgewählt ist.

12. Zusammensetzung nach Anspruch 8, **dadurch gekennzeichnet, dass** die organische Verbindung ein synthetisches Oligomer oder Polymer ist, das mindestens einen aromatischen Kern oder eine polare Gruppe besitzt und insbesondere ausgewählt ist aus Polyvinylpyrrolidon-Derivaten (PVP), Polyvinylalkohol-Derivaten (PVA), Polyacryl-Derivaten in ihrer polymeren und copolymeren Form, Polymethacrylsäure-Derivaten (PMAA), Polyvinyloxazolidon- (PVO) und Polyvinylmethyloxazolidon-Derivaten (PVMO), Polyvinyloxazolin-Derivaten.

13. Zusammensetzung nach einem der vorhergehenden Ansprüche, **dadurch gekennzeichnet, dass** sie in Form einer Emulsion vorliegt.

14. Zusammensetzung nach einem der vorhergehenden Ansprüche, **dadurch gekennzeichnet, dass** die Emulsion vom Typ einer direkten oder inversen Emulsion vorliegt.

15. Zusammensetzung nach einem der Ansprüche 13 oder 14, **dadurch gekennzeichnet, dass** die Emulsion vom Typ einer Wasser-in-Öl- oder Öl-in-Wasser- oder Mehrfach-Emulsion vorliegt.

16. Zusammensetzung nach einem der Ansprüche 13 bis 15, **dadurch gekennzeichnet, dass** sie ein emulgierendes Mittel und gegebenenfalls ein coemulgierendes Mittel in einem Anteil von kleiner als 30 Gew.-% und insbesondere kleiner als 20 Gew.-% und spezieller kleiner als 10 Gew.-% umfasst.

17. Zusammensetzung nach einem der Ansprüche 13 bis 15, **dadurch gekennzeichnet, dass** sie frei ist von emulgierenden Mitteln und coemulgierenden Mitteln.

18. Zusammensetzung nach einem der vorhergehenden Ansprüche, **dadurch gekennzeichnet, dass** sie eine kontinuierliche Fettphase umfasst.

19. Zusammensetzung nach einem der vorhergehenden Ansprüche, **dadurch gekennzeichnet, dass** sie wasserfrei ist.

20. Zusammensetzung nach einem der vorhergehenden Ansprüche, **dadurch gekennzeichnet, dass** die Fettphase weiterhin mindestens einen bei Umgebungstemperatur und Atmosphärendruck flüssigen Fettkörper und gegebenenfalls mindestens einen bei Umgebungstemperatur und Atmosphärendruck festen Fettkörper umfasst.

21. Zusammensetzung nach Anspruch 20, **dadurch gekennzeichnet, dass** der bei Umgebungstemperatur und Atmosphärendruck flüssige Fettkörper mindestens ein nicht flüchtiges Öl umfasst.

22. Zusammensetzung nach Anspruch 21, **dadurch gekennzeichnet, dass** das nicht flüchtiges Öl ausgewählt ist aus Kohlenwasserstoffölen tierischer Herkunft, pflanzlichen Kohlenwasserstoffölen, linearen oder verzweigten Kohlenwasserstoffen mineralischer oder synthetischer Herkunft, Syntheseethem mit 10 bis 40 Kohlenstoffatomen, Syntheseestern, Polyolestern, bei Umgebungstemperatur flüssigen Fettalkoholen mit 12 bis 26 Kohlenstoffatomen, höheren Fettsäuren, silikonierten Ölen vom Typ der Polymethylsiloxane (PDMS) und ihren Gemischen.

23. Zusammensetzung nach einem der Ansprüche 20 bis 22, **dadurch gekennzeichnet, dass** der flüssige Fettkörper mindestens ein flüchtiges Öl umfasst, das aus Kohlenwasserstoffölen mit 8 bis 16 Kohlenstoffatomen und Ölen von flüchtigen Silikonen ausgewählt ist.

24. Zusammensetzung nach einem der vorhergehenden Ansprüche, **dadurch gekennzeichnet, dass** sie eine Ölphase in einem Anteil umfasst, der von 1 bis 80 % und insbesondere von 1 bis 50 % variiert, bezogen auf das Gesamtgewicht der Zusammensetzung.

25. Zusammensetzung nach einem der vorhergehenden Ansprüche, **dadurch gekennzeichnet, dass** die mit Wasser nicht mischbare organische Phase ein oder mehrere organische Lösungsmittel einschließt, deren Mischbarkeit in Wasser kleiner oder gleich 50 % Gew.-% bei 25 °C ist.

26. Zusammensetzung nach einem der vorhergehenden Ansprüche, **dadurch gekennzeichnet, dass** das Lösungsmittel ausgewählt ist aus kurzkettigen Estern mit 4 bis 8 Kohlenstoffatomen insgesamt, wie Ethylacetat, Propylacetat, Butylacetat, Arylacetat, Isopentylacetat, und ihren Gemischen.

27. Zusammensetzung nach einem der vorhergehenden Ansprüche, **dadurch gekennzeichnet, dass** sie mindestens ein Farbmittel umfasst.

28. Zusammensetzung nach dem vorhergehenden Anspruch, **dadurch gekennzeichnet, dass** das Farbmittel aus wasserlöslichen Farbstoffen, fettlöslichen Farbstoffen, Pigmenten und Lacken ausgewählt ist.

29. Zusammensetzung nach Anspruch 27 oder 28, **dadurch gekennzeichnet, dass** sie 0,05 Gew.-% bis 20 Gew.-% und insbesondere 0,1 bis 15 Gew.-% und speziell 0,5 bis 10 Gew.-% Farbmittel umfasst.

30. Zusammensetzung nach dem vorhergehenden Anspruch, **dadurch gekennzeichnet, dass** sie durchscheinend oder transparent ist und/oder dazu in der Lage ist, eine durchscheinende oder transparente Ablagerung zu ergeben.

31. Verwendung von exfolierten Phyllosilikaten, wie nach einem der Ansprüche 1 bis 12 definiert, als Mittel zur Strukturierung der flüssigen Fettphase oder der organischen Phase einer Nagellack-Zusammensetzung, wobei die Phase mit Wasser nicht mischbar ist und ein oder mehrere organische Lösungsmittel einschließt, deren Mischbarkeit in Wasser bei 25 °C kleiner oder gleich 50 Gew.-% ist.

32. Verwendung nach dem vorhergehenden Anspruch, **dadurch gekennzeichnet, dass** die Zusammensetzung eine Emulsion ist.

33. Verwendung nach Anspruch 31 oder 32, **dadurch gekennzeichnet, dass** die Zusammensetzung durchscheinend oder transparent ist und/oder dazu in der Lage ist, eine durchscheinende oder transparente Ablagerung zu ergeben.

34. Verfahren zum Übermalen der Phaneren, umfassend das Aufbringen auf die Phaneren von mindestens einer Schicht einer Zusammensetzung wie nach einem der Ansprüche 1 bis 30 definiert.

35. Synthetischer übermalter Träger, auf dem überall oder auf einem Teil seiner Oberfläche eine Zusammensetzung wie nach einem der Ansprüche 1 bis 30 definiert vorhanden ist.
